# EUROPEAN PATENT SPECIFICATION

(11) **EP 3 592 161 B1**
(45) Date of publication and mention of the grant of the patent: **27.12.2023**
(21) Application number: 18750599.5
(22) Date of filing: 07.02.2018
(51) Int. Cl.: A41D 19/015, A61B 42/10, A61B 42/40, A61B 42/50, A61B 50/20, B65D 83/08, B65D 85/18

(54) **PACKING SYSTEM FOR MEDICAL DISPOSABLE GLOVES WITH THE METHOD FOR EXTERNAL EXTRACTION REDUCING CONTAMINATION**
VERPACKUNGSSYSTEM FÜR MEDIZINISCHE EINWEGHANDSCHUHE MIT EINEM VERFAHREN ZUR EXTERNEN EXTRAKTION ZUR VERRINGERUNG VON VERUNREINIGUNGEN
SYSTÈME DE CONDITIONNEMENT POUR GANTS MÉDICAUX JETABLES COMPRENANT UN PROCÉDÉ D'EXTRACTION EXTERNE RÉDUISANT LA CONTAMINATION

(30) Priority: 13.02.2017 US 201762600148 P; 11.05.2017 PL 42156717
(43) Date of publication of application: 15.01.2020
(73) Proprietor: Dorothy Ressel Intellectual Properties, 34-143 Lanckorona (PL)
(72) Inventor: DOROTA, Ressel, AZ 85119 (US)
(74) Representative: Krekora, Magdalena
(86) International application number: PCT/US2018/017235
(87) International publication number: WO 2018/148288

(56) References cited:
- GB-A- 2 451 450
- GB-A- 2 510 428
- GB-A- 2 510 428
- GB-A- 2 528 242
- GB-A- 2 528 242
- GB-B- 2 519 839
- US-A- 4 677 697
- US-A1- 2003 116 580
- US-A1- 2012 298 689
- US-A1- 2014 061 220
- US-A1- 2016 143 490
- US-A1- 2016 143 490

## Description

This disclosure relates to a method of packing and dispensing disposable gloves and includes the new container and the system for carrying out this method.

Disposable gloves are used in many fields and it is in medicine and diagnostics that the necessity of preserving them in an aseptic state is particularly essential. Studies carried out at American hospitals and clinics show that on average at least 50% of disposable gloves used by personnel are contaminated and in the case of gloves extracted from boxes placed close to water sources this share reaches 75%. Use of gloves often gives a false sense of safety which also results in the personnel not recognising the necessity of careful disinfection of the hands or the necessity of careful drying of the hands before inserting hands into the boxes that contain the gloves.

From the description of Patent US6708841B2 there is known a wall-mountable glove dispenser into which a box of gloves is placed which enables dispensing of gloves.

From the description of Patent US7063233B2 there is known a glove dispenser which enables one-off dispensing of a selected number of gloves.

From the description of Patent US5044494A there is known a packaging case for packing either left-hand or right-hand gloves.

From the description of Patent US7699189B2 there is known a glove dispenser in which gloves are tilted towards the opening through which they are dispensed to facilitate the extracting of each glove. The dispenser is configured in such a manner that a portion of the glove protrudes from the opening making it easier to grip and pull out.

From the description of Patent US4844293A there is known a box for thin disposable gloves where the design of the box makes it possible to pull out single gloves.

From the description of Patent US5655682 there is known a system of dispensing products consisting of a package of disposable plastic sheets that can be removed from the package one at a time to unpack the articles, e.g. gloves.

From the description of Patent US5816440 there is known a system of dispensing disposable gloves where it is only the cuff of a single glove that protrudes from the dispensing hole and extracting one glove results in pulling the cuff of the next glove outside.

From the description of Patent GB2495023 there is known a glove dispenser where gloves are pushed upwards to facilitate taking them out of the pack. Gloves are arranged in such a manner that they can be taken out by touching only the cuff.

GB 2 510 428 A discloses a system of dispensing disposable gloves.

None of the solutions described above protect the packed gloves in a sufficient manner nor is it easy to implement packaging in mass production.

The invention is defined in the independent claim and other embodiments are listed in the dependent claims. No methods are claimed.

This invention provides and shows that it is possible to develop a solution with less disadvantages of the systems known from the prior art and which can more easily accommodate automated mass production packaging.

This new method of protecting disposable gloves against contamination is special and characterized in that at least one glove is placed in a container of a waterproof material, preferrably of a plastic, which then closes tightly and where the cuff of at least one glove is positioned near the opening hole for extracting gloves.

The internal surface of the container is coated with a biocidal substance.

The container for at least one glove is attached to a rack tilted at an angle relative to the ground where the opening hole for extracting gloves is oriented downwards and towards the ground.

The container for disposable gloves according to this invention is special and characterized in that it has an opening for extracting gloves where the opening is protected with a tight closing seal mechanism and at least one glove is placed in the container in such a manner that its cuff is near of the opening used for extracting gloves.

The area of the opening hole for extracting gloves is coated with a biocidal substance. And the inside of the container is coated with a biocidal substance.

The glove container is made of a waterproof material which is preferably of a plastic.

The container is in the shape of a bag with permanent seals at the top and bottom parts and the exterior portion of the seals are perforated to enable attaching the container to the rack.

The dispensing system for disposable gloves according to this invention is special and characterized in that it comprises at least one container with at least one glove as well as a rack for attaching the container. The container is in the shape of a bag sealed at the top and bottom and is attached to the rack where both the top part and the bottom part of the bag are attached to the rack such that the bag is stretched between the fixing points. The container has a tightly closed and sealed opening hole, for extracting gloves, and where the glove cuff is in the immediate proximity of the opening hole.

The container attached to the rack is tilted in relation to the ground at an angle less than 90 degrees horizontal to the ground. It is advantageous when this angle is in the range between 30 and 50 degrees.

This invention has the opening hole for glove extracting oriented downwards.

The pulling of gloves from the container does not require inserting the hand inside the container and the force of gravity both facilitates extracting the glove and helps prevent impurities and water from getting inside the container and thus the risk of contaminating the gloves inside the container is reduced.

In one container there can be one glove or there can be more than one glove. The cuff of each glove is placed in the immediate proximity of the opening hole for extracting gloves so that the user extracting a glove only grips the cuff and does not touch the other parts of the glove. The opening hole for extracting gloves is sealable. The closing mechanism can be any closing mechanism known, such as glue-covered film, enabling the hole to be opened and closed multiple times.

The system can comprise more than one container stretched between the racks. The container is inclined to the horizontal at an angle less than 90 degrees. It is most advantageous when the angle is between 30 and 50 degrees. Such an inclination is optimal as it facilitates the pulling of gloves from the container. In this system, all gloves can be pulled out one at a time in a manner that reduces the risk of the glove being contaminated. The person extracting a glove only touches it with a bare hand at the cuff and after donning it on they can extract another glove and done it on the other hand. Gloves should be packed in such a manner that the cuff of each one of them should be at the height of the opening hole for extracting them. For gloves that differ from each other depending on whether they are intended for the right or the left hand, they should be packed alternately, or separate containers for right-hand gloves and for left-hand gloves can be used.

The container being made of plastic is designed to enable the coating of its internal surfaces, including the area of the opening hole, with biocidal substances, which additionally reduces the hazard of the gloves getting contaminated by microbes.

The rack for attaching containers is comprised of hooks on which to hang the containers. For this reason containers should have perforations where the hooks can be placed. It is also possible to use other types of attachments such as clips or clamps. The attachments (including hooks) are connected to the rack by means of a spring or another elastic connector. The rack for containers can be a rigid structure and can also be foldable.

The rack can be mounted on a wall or on a special stand. The rack is attached in such a manner that the container should be at an angle less than 90 degrees relative to the ground.

This glove removal and glove container together form a solution that enables more safe dispensing of disposable gloves in a manner that helps protect them from contamination. Due to this the risk of hospital infections, and/or diagnostic errors, resulting from glove contamination will be reduced.

The object of the invention is shown in example drawings where Fig. 1 presents the container for gloves, Fig. 2 presents the container for gloves attached to the rack, Fig. 3 presents the manner of extracting gloves from the container, and Fig. 4 presents a foldable rack for containers.

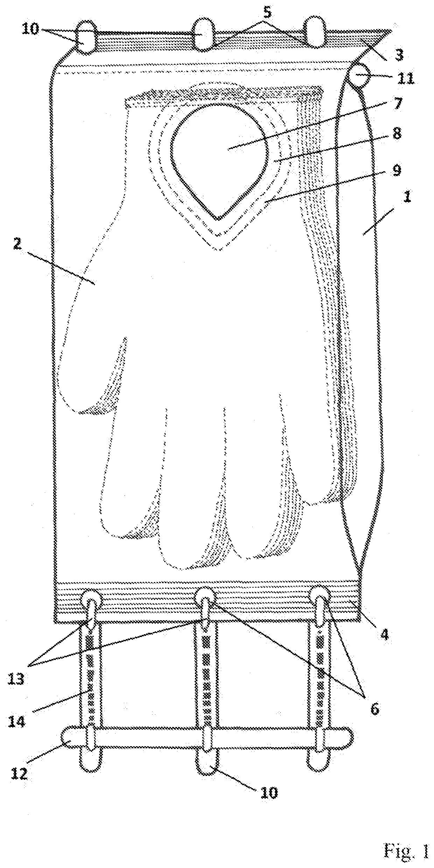

Example, using Fig 1 as reference:
The container (1) for disposable gloves (2) is made of a waterproof material which is advantageously of a plastic. Inside the container there are disposable gloves (2). The top part of the container (1) is sealed by means of a permanent seal (3) and the bottom part of the container (1) is also sealed by means of a permanent seal (4). These permanent seals (3 and 4) have perforations (5 and 6). In the top part of the container (1) there is an opening hole (7) for extracting the glove(s) (2). The external surface of the container (1), in the immediate proximity of the opening hole (7), is coated with glue (8) where to film (9) covering and securing the opening hole (7) is attached. Glue (8) is a type of glue which enables film (9) to be opened and closed multiple times. Vertical pillars (10) of the rack have the container (1) attached in such a manner that the ends of the poles are placed in the perforations (5). Container (1) rests on the horizontal bracket (11). At the bottom part of the rack there is a horizontal bracket (12) connected to the pillars (10). There are hooks (13) attached to bracket (12) through springs (14). The hooks (13) pass through the perforations (6).

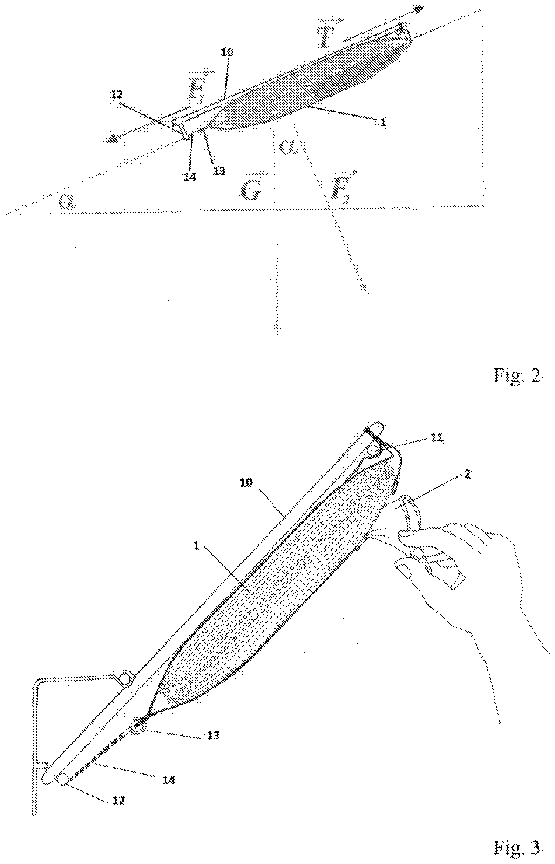

At Fig 2 and 3 please see that the container (1) is attached to pillars (10) which are tilted at an angle (α) relative to the ground. The top part of the container (1) is attached to the pillars (10) and the bottom part (of the container) is attached to the bracket (12) by means of a system of hooks (13) with springs or elastic (14). The force of gravity (G) helps a glove to move freely downwards. The container is stretched on the rack and it is acted on by a force of tension (T). When a glove (2 Fig 3) is pulled out of the container the container is additionally acted on by a force (F2 Fig 2) and at the same time a force (F1) generated by the springs (14).

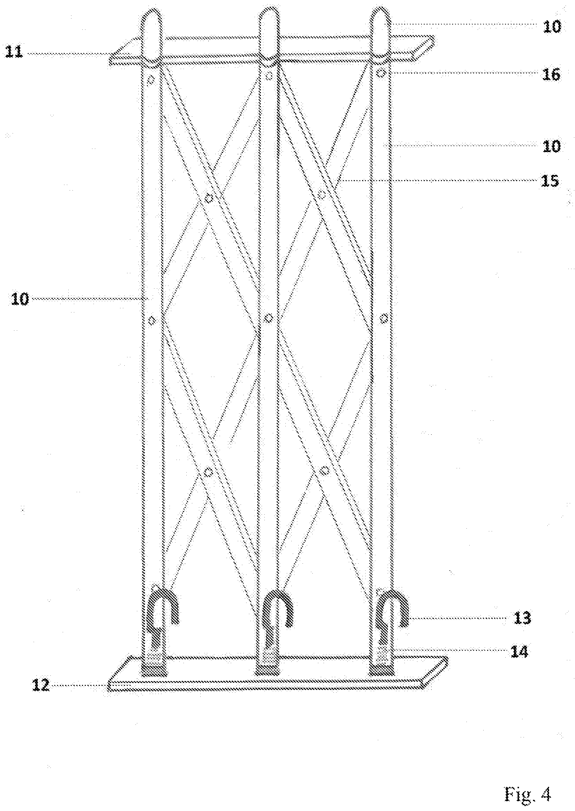

At Fig 4 please see that the rack for attaching containers consists of vertical poles (10) and horizontal brackets (11 and 12). The bottom horizontal bracket (12) has hooks (13) attached to it on springs or elastic (14). The rack is reinforced with diagonal brackets (15) which are connected to the poles (10) by means of hardware connectors (16).

## Claims

1. System of dispensing disposable gloves comprising at least one container (1) with at least one glove (2) and a rack for attaching the container, where the container is in the shape of a bag sealed at the top and at the bottom, and is attached to the rack, **characterized in that**, both the top part and the bottom part of the container (1) are attached to the rack such that the container (1) is stretched between fixing points (10, 13), and the container (1) has a tightly closed and sealed opening hole (7) for extracting gloves (2) and where a glove cuff (2) of at least one glove (2) is located in the immediate proximity of the opening hole (7) for extracting gloves (2).

2. System according to claim 1, **characterized in that**, the container (1) attached to the rack is tilted relative to the ground at an angle (α) that is less than 90 degrees.

3. System according to claim 2, **characterized in that**, the angle (α) is between 30 and 50 degrees.

4. System according to claim 1 or 2 or 3, **characterized in that**, the opening hole (7) for extracting gloves (2) is oriented downwards towards the ground.

5. System according to any of preceding claims, **characterized in that**, a region of the opening hole (7), for extracting gloves (2), is coated with a biocidal substance.

6. System according to claim 5, **characterized in that**, an interior of the container (1) is coated with a biocidal substance.

7. System according to claim 5 or 6, **characterized in that**, the container (1) is made of a waterproof material, preferably and advantageously of a plastic.

8. System according claim 5 or 6 or 7, **characterized in that**, the opening hole (7) for extracting gloves (2) is protected with a closing mechanism (9) which is configured to be opened and closed multiple times.

## Patentansprüche

1. System zur Ausgabe von Einweghandschuhen, bestehend aus mindestens einem Behälter (1) mit mindestens einem Handschuh (2) und einem Gestell zum Befestigen des Behälters, wobei der Behälter die Form eines Beutels hat, der oben und unten verschlossen ist, und an dem Gestell befestigt ist, **dadurch gekennzeichnet, dass** sowohl der obere Teil als auch der untere Teil des Behälters (1) so an dem Gestell befestigt sind, dass der Behälter (1) zwischen Befestigungspunkten (10, 13) gespannt ist, und der Behälter (1) ein dicht geschlossenes und abgedichtetes Öffnungsloch (7) zur Entnahme von Handschuhen (2) aufweist und wobei sich eine Handschuhstulpe (2) mindestens eines Handschuhs (2) Handschuhs (2) in unmittelbarer Nähe des Öffnungslochs (7) zur Entnahme von Handschuhen (2).

2. System nach Anspruch 1, **dadurch gekennzeichnet, dass** der am Gestell befestigte Behälter (1) relativ zum Boden in einem Winkel (α) geneigt ist. gegenüber dem Boden in einem Winkel (a) von weniger als 90 Grad geneigt ist.

3. System nach Anspruch 2, **dadurch gekennzeichnet, dass** der Winkel (α) zwischen 30 und 50 Grad beträgt.

4. System nach Anspruch 1 oder 2 oder 3, **dadurch gekennzeichnet, dass** das Öffnungsloch (7) zum Herausziehen der Handschuhe (2) nach unten zum Boden hin ausgerichtet ist.

5. System nach einem der vorhergehenden Ansprüche, **dadurch gekennzeichnet, dass** ein Bereich des Öffnungslochs (7) zum Herausziehen der Handschuhe (2) mit einer bioziden Substanz beschichtet ist.

6. System nach Anspruch 5, **dadurch gekennzeichnet, dass** ein Innenraum des Behälters (1) mit einer bioziden Substanz beschichtet ist.

7. System nach Anspruch 5 oder 6, **dadurch gekennzeichnet, dass** der Behälter (1) aus einem wasserdichten Material, vorzugsweise und vorteilhaft aus einem Kunststoff, hergestellt ist.

8. System nach Anspruch 5 oder 6 oder 7, **dadurch gekennzeichnet, dass** das Öffnungsloch (7) zur Entnahme von Handschuhen (2) mit einem Verschlussmechanismus (9) gesichert ist, der zum mehrfachen Öffnen und Schließen ausgeführt ist.

## Revendications

1. Système de distribution de gants jetables comprenant au moins un conteneur (1) avec au moins un gant (2) et un support pour fixer le conteneur, dans lequel le conteneur a la forme d'un sac scellé en haut et en bas, et est fixé au support, **caractérisé en ce que** à la fois la partie supérieure et la partie inférieure du conteneur (1) sont fixées au support de telle sorte que le conteneur (1) est tendu entre les points de fixation (10, 13), et que le conteneur (1) possède un trou d'ouverture (7) hermétiquement fermé et scellé pour extraire les gants (2) et qu'une manchette de gant (2) d'au moins un gant (2) est située à proximité immédiate du trou d'ouverture (7) pour extraire des gants (2).

2. Système selon la revendication 1, **caractérisé en ce que** le conteneur (1) fixé au support est incliné par rapport au sol selon un angle (α) inférieur à 90 degrés.

3. Système selon la revendication 2, **caractérisé en ce que** l'angle (α) est compris entre 30 et 50 degrés.

4. Système selon la revendication 1 ou 2 ou 3, **caractérisé en ce que** le trou d'ouverture (7) pour l'extraction de gants (2) est orienté vers le bas en direction du sol.

5. Système selon l'une quelconque des revendications précédentes, **caractérisé par le fait qu'**une région du trou d'ouverture (7) pour l'extraction de gants (2) est enduite d'une substance biocide.

6. Système selon la revendication 5, **caractérisé par le fait qu'**un intérieur du conteneur (1) est enduit d'une substance biocide.

7. Système selon la revendication 5 ou 6, **caractérisé par le fait que** le conteneur (1) est constitué d'un matériau imperméable, de préférence et avantageusement d'une matière plastique.

8. Système selon la revendication 5 ou 6 ou 7, **caractérisé par le fait que** le trou d'ouverture (7) pour l'extraction de gants (2) est protégé par un mécanisme de fermeture (9) qui est configuré pour être ouvert et fermé plusieurs fois.
